(19) Europäisches Patentamt
European Patent Office
Office européen des brevets

(11) Publication number : 0 539 167 A2

# (12) EUROPEAN PATENT APPLICATION

(21) Application number : 92309583.0

(22) Date of filing : 20.10.92

(51) Int. Cl.$^5$ : A61K 47/48

(30) Priority : 21.10.91 US 779798

(43) Date of publication of application :
28.04.93 Bulletin 93/17

(84) Designated Contracting States :
AT BE CH DE DK ES FR GB GR IT LI LU NL SE

(71) Applicant : ORTHO PHARMACEUTICAL CORPORATION
U.S. Route 202 P.O. Box 300
Raritan New Jersey 08869-0602 (US)

(72) Inventor : Wright, David E.
10827 Ashlar Place
San Diego, CA 92131 (US)

(74) Representative : Mercer, Christopher Paul et al
Carpmaels & Ransford 43, Bloomsbury Square
London WC1A 2RA (GB)

## (54) Peg imidates and protein derivatives thereof.

(57) The present invention provides methods and compounds for modifying proteins, peptides and organic compounds with free amino group(s) with an imidate derivative of PEG or related water-soluble organic polymers. Novel imidate derivatives of PEG and other water-soluble polymers are provided for.

The water-soluble polymer modifying reagents of the subject invention included imidates of polyethylene glycol, polypropylene glycol, polyoxyethylated polyol, heparin, heparin fragments, dextran, polysaccharides, polyamino acids, polyvinyl alcohol, and other water-soluble organic polymers also is included.

An advantage of PEG-imidates is that on reaction with the amino groups of proteins the resulting derivative still preserves the positive charge and displaces the positive charge away from the water-soluble polymer backbone.

Also provided for are polypeptides of interest derivatized by water-soluble polymer imidates.

EP 0 539 167 A2

Jouve, 18, rue Saint-Denis, 75001 PARIS

This invention relates to modified water-soluble polymers with a terminal imidate group and polypeptide molecules modified by such molecules.

Protein and other similar organic molecules may be chemically modified by conjugation to water-soluble organic polymers such as polyethylene glycol (PEG). The production of such protein conjugates is of interest because of the desirable properties conferred on polypeptides by the attachment of the water-soluble polymers. These desirable properties include increased solubility in aqueous solutions, increased stability during storage, reduced immunogenicity, increased resistance to enzymatic degradation, compatibility with a wider variety of drug administration systems, and increased in vivo half-life. These properties that are brought about by the derivatization of polypeptides with PEG or other water-soluble polymers are especially of interest when the polypeptide is to be used as a therapeutic agent injected into the body or when the polypeptide is to be used in assays, usually immunoassays, for the detection and/or quantification of a compound of interest.

The attachment of reporter groups, ligands, etc. to proteins usually proceeds through the primary amino group on the side chains of the lysine residues. Several PEG-derivatives have been synthesized that specifically couple with lysine residues. The hydroxyl end groups of PEG are typically converted to reactive functional groups capable of covalent attaching PEG to a protein of interest. These PEG-derivatives include: 2 (alkoxypolyethyleneglycoxy)-4, 6-dichlorotriazine [Abuchowski, A., Van Es, T., Palczuk, N.C., and Davis, F.F. (1977) J. Biol. Chem. 252, 3578-3581]; 2-(alkoxypolyethylene glycoxy)-S-carboxamidomethyldithiocarbonate [King, T.P., and Weiner, C. (1980) Int. J. Peptide Protein Res. 16, 147-155]; 2-(alkoxypolyethyleneglycoxy)-N-succinimidyl succinate [Abuchowski, A., Kazo, G.M., Verhoest, C., Van Es, T., Kafkewitz, D., Viau, A., and Davis, F. (1984) Cancer Biochem. Biophys. 7, 175-186]; 2-(alkoxypolyethyleneglycoxycarboxyimidazole [Beauchamp, C.O., Gonias, S.L., Menapace, D.P., and Pizzo, S.V. (1983) Anal. Biochem. 131, 25-33]; 2-(alkoxypolyethyleneglycoxy)-2, 4, 5-trichlorobenzene [Versonese, F.M., Largajolli, R., Boccu, E., Benassi, C.A., and Schiavon, O. (1985) Applied Biochem. Biotech 11, 141-152]; 2-(alkoxypolyethyleneglycoxy)-4-nitrobenzene [Versonese, F.M., Largajolli, R., Boccu, E., Benassi, C.A., and Schiavon, O. (1985) Applied Biochem. Biotech 11, 141-152]; 2-(alkoxypolyethyleneglycoxy)-2, 2, 2-trifluoroethane [Delgado, C., Patel, J.N., Francis, G.E., and Fisher, D. (1990) Biotech. Applied Biochem. 12, 119-128]; 2-(alkoxypolyethylenealdehyde [Andrews, B.A., Head, D.M., Dunthorne, P., and Asenjo, J.A. (1990) Biotech. Tech. 4, 49-54]; 2-alkoxypolyethyleneglycoxymethylepoxide [Andrews, B.A., Head, D.M., Dunthorne, P., and Asenjo, J.A. (1990) Biotech. Tech. 4, 49-54].

Alkyl imidates have been used frequently for protein modification. [For a review see Hunter, M.J., and Ludwig, M.L. (1972) Methods Enzymol. 25, 585-596.] Alkyl imidates react specifically with alpha and epsilon amino groups yielding amidines which are stronger bases than the parent amines. The positive charge of the original protein amino group is retained although the positive charge has been shifted by a few atoms. A variety of functional groups (phenyl, phenolic, sulfhydryl, fluorescent, hydrophobic, radiolabel, spin label, photoaffinity label, etc.) can be attached to imidoesters and in this manner introduced into a protein. An advantage of imidoesters over active ester coupling or active carbamate coupling lies in the preservation of the positive charge of the lysine group being modified.

Advantages of coupling water-soluble polymers, especially polyethylene glycol, to proteins have been well documented and include the following: increased solubility of the conjugated protein as compared with the native protein at physiological pH when native protein is insoluble or only partially soluble at physiological pH, a decrease in the immune response generated by the native protein, an increased pharmokinetic profile, an increased shelf-life, and an increased biological half-life.

A number of proteins have been modified by PEG. [For a review see Inada, Y., Yoshimoto, T. Matsushima, A., and Saito, Y. (1986) Trends Biotechnol. 4: 68-73.] A number of patent applications also have issued or published in this area as listed below: U.S. Pat. No. 4,179,337; U.S. Pat. No. 4,609,546; U.S. Pat. No. 4,261,973; U.S. Pat. No. 4,055,635; U.S. Pat. No. 3,960,830; U.S. Pat. No. 4,415,665; U.S. Pat. No. 4,412,989; U.S. Pat. No. 4,002,531; U.S. Pat. No. 4,414,147; U.S. Pat. No. 3,788,948; U.S. Pat. No. 4,732,863; U.S. Pat. No. 4,745,180; EP No. 152,847; EP No. 98,110 published January 11, 1984; JP No. 5,792,435. The above patents and patent publications also describe the use of other water-soluble polymer protein modifying reagents including but not restricted to polypropylene glycol (PPG), polyoxyethylated polyol (POP), heparin, heparin fragments, dextran, polysaccharides, polyamino acids including proline, polyvinyl alcohol (PVA), and other water-soluble organic polymers.

Several limitations exist with respect to which polypeptides may be conjugated to water-soluble polymers and the extent to which the polypeptides can be modified.

Different water-soluble polymer reagents vary with respect to the functional groups that provide for coupling to amino acid residues in polypeptides of interest. The term water-soluble polymer reagent as used hereinafter refers to a water-soluble polymer modified so as to contain a functional group that provides for the conjugation of the water-soluble polymer to a polypeptide. Specific functional groups provide for the coupling of water-soluble polymers to specific amino acid residues. It is of interest to provide for water-soluble polymer

reagents that may be coupled to amino acid residues that are not subject to modification by known water-soluble polymer reagents. By providing for water-soluble polymer reagents that may be coupled to amino acid residues not previously subject to coupling, it becomes possible to conjugate water-soluble polymers to proteins without substantially adversely affecting the biological activity of proteins that would be adversely affected through coupling at other amino acid residues.

Another limitation on modifying polypeptides by the coupling of water-soluble polymers is charge alteration caused by the coupling reaction. Changes in charge, either the introduction of charge to an uncharged residue or vice versa, on an amino acid residue may adversely affect the biological activity of a protein by several mechanisms including the disruption of the tertiary structure and the destruction of active sites. Since such changes in charge are cumulative, it is apparent that charge altering coupling reactions may limit the amount of derivatization possible without substantially impairing the function of the polypeptide. Thus it is of interest to provide water-soluble polymer reagents that are capable of coupling to amino acid residues without altering the charge present on the residue.

The present invention provides methods and compounds for modifying proteins, peptides and organic compounds having free amino group(s) with an imidate derivative of PEG or an imidate derivative of another structurally related water-soluble organic polymers. Novel imidate derivatives of PEG and other water-soluble polymers are provided. One or more water-soluble polymer imitate esters may be coupled to individual polypeptides or similar organic molecules. Another aspect of the subject invention is to provide for proteins modified by the covalent attachment of the imidate water-soluble polymer derivatives.

An advantage of imidate water soluble polymer derivatives is that on reaction with the amino groups of proteins the resulting protein derivative preserves the positive charge. Imidate coupling also displaces the positive charge away from the water-soluble polymer backbone, which may make the positive charge more accessible for binding to receptor, targeted proteins or ligands. Thus modification of proteins by watersoluble polymer imidate derivatives avoids the loss of positive charge seen with the commonly used PEG-modifying reagents using couplers such as N-hydroxysuccinimide esters, cyanuric chloride, and active carbamates.

The water-soluble polymer reagents of the subject invention include imidates of polyethylene glycol homopolymers, polypropylene glycol homopolymers, copolymers of ethylene glycol with propylene glycol, wherein said homopolymers and copolymers are unsubstituted or substituted at one end with an alkyl group, polyoxyethylated polyols, polyvinyl alcohol, polysaccharides, polyvinyl ethyl ethers, and α,β-Poly [(2-hydroxyethyl)-DL-aspartamide] and other water-soluble organic polymers.

Polypeptides of interest for water-soluble polymer derivatization by the subject water-soluble polymer imidates include hormones, lymphokines, cytokines, growth factors, enzymes, vaccine antigens, and antibodies. Water-soluble polymer derivatization of erythropoietin (EPO), especially human erythropoietin is of particular interest.

Another aspect of the invention is EPO derivatized with water-soluble polymer tresyl and succinimide esters.

The subject invention provides novel polypeptide modifying reagents that are imidate derivatives of water-soluble polymers such as PEG, i.e, polyethylene glycol. The molecules of the subject invention may be used to covalently attach a variety of water-soluble polymers to polypeptides of interest. Another aspect of the subject invention is to provide polypeptides modified by the reagent molecules, i.e., the subject water-soluble polymer imidates, so as to be covalently bonded to one or more water-soluble polypeptides.

In general, the formula of the compounds useful for coupling water-soluble polymers to polypeptides is as follows:

$$\text{(I)}\qquad P-\overset{\overset{\displaystyle OCH_3}{|}}{C}=NH_2^{+}Cl^{-}$$

and

$$\text{(II)}\qquad P-NH-(CH_2)_m-\overset{\overset{\displaystyle OCH_3}{|}}{C}=NH_2^{+}Cl^{-}$$

P represents a water-soluble organic polymer. Water-soluble organic polymers of interest have hydroxy

groups appended to the polymer backbone and may be selected from known water-soluble polymers including but not limited to: (a) dextran and dextran derivatives, including dextran sulfate, P-amino cross linked dextrin, and carboxymethyl dextrin (b) cellulose and cellulose derivatives, including methylcellulose and carboxymethyl cellulose (c) starch and dextrines, and derivatives and hydroylactes of starch (d) polyalklyene glycol and derivatives thereof, including polyethylene glycol, methoxypolyethylene glycol, polyethylene glycol homopolymers, polypropylene glycol homopolymers, copolymers of ethylene glycol with propylene glycol, wherein said homopolymers and copolymers are unsubstituted or substituted at one end with an alkyl group (e) heparin and fragments of heparin, (f) polyvinyl alcohol and polyvinyl ethyl ethers, (g) polyvinylpyrrolidone, (h) α,β-Poly[(2-hydroxyethyl)-DL-aspartamide, and (i) polyoxyethylated polyols. Preferably, the water-soluble polymer P is selected from dextran and dextran derivatives, dextrine and dextrine derivatives, polyethylene glycol and derivatives thereof. Most preferably, the water-soluble polymer P is selected from polyethylene glycol and derivatives thereof, the monomethyl ether of polyethylene glycol being particularly preferred (so as to avoid crosslinking between proteins). When polypeptides modified by the water-soluble polymer reagents of the subject invention are to be used as pharmaceuticals, polymer P should be non-toxic.

In formula II, m is in the range of about 1 to 20, the range of 1 to 10 being particularly preferred.

In addition to the molecules of formula I, the subject invention also includes polypeptides modified by reaction with the molecules of formula I and II.

Polypeptides modified by the water-soluble polymer reagents of formulas (I) and (II) may be represented by formulas (III) and (IV), respectively.

(III)

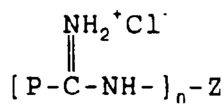

and

(IV)

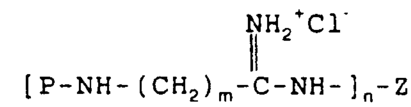

wherein P is a water-soluble polymer as previously described. Z represents a polypeptide as described above and containing at least one primary amine containing residue and n represents a number in the range 1 to x, where x is one more than the number of lysine residues present in polypeptide Z. In formula IV, m is the range of about 1 to 20, the range of 1 to 10 being particularly preferred. Water-soluble polymer P is covalently joined to epsilon amino groups of the lysine residues and/or the terminal amino group on the polypeptide at the site of coupling. Although polypeptides may be modified by the coupling of up to x water-soluble polymers per polypeptide molecule, it is usually desirable to modify a given polypeptide by less than x water-soluble polymer molecules. It may be undesirable to derivatize a polypeptide with the maximum number of water-soluble polymers, i.e., x molecules, because increasing the number of water-soluble polymers per molecule of polypeptide may diminish biological activities possessed by the unmodified polypeptide.

An important advantage of the subject invention is that polypeptides may be modified by the attachment of water-soluble polymers without substantially reducing the biological activity of the polypeptide, or reducing the biological activity to a lesser extent than the biological activity would be reduced by the attachment of the same water-soluble polymers to lysine residues through compounds other than the imidoesters of the subject invention.

The term "biological activity" includes enzymatic activity, the ability to bind to receptors (including antibodies), the ability to bind ligands, the ability to induce an immune response, and the like.

By the term "antibodies," it is intended to include both polyclonal and monoclonal antibodies with natural immunoglobulin sequences, synthetic antibody derivatives, and the like; antibodies may be modified so as to be joined to any of a variety of labels, fluorescent, radioactive, enzymatic, biotin/avidin or the like. Synthetic antibody derivatives include natural immunoglobulin sequences that have been mutated and selected for altered binding specificity, various immunoglobulin gene derived polypeptides, typically single chain, produced by genetically modified bacteria, antibodies modified so as to contain modified constant regions and the like;

a review of such synthetic antibody derivatives based on the principles of antibody formation is provided in Winter and Milstein, Nature, 349: 293-299 (1991).

An advantage of the subject invention is that polypeptides modified by the compounds of formulas (I) and (II) may then retain a greater degree of their biological activity than when the same polypeptide is modified to the same degree by joining water-soluble polymers to polypeptides employing other esters. This advantage may be a consequence of the preservation of the positive charge on the lysine residues modified by the imidoesters and displacement of the positive charge away from the soluble polymer backbone. Thus, the subject invention provides for modified polypeptides that possess the advantages associated with the conjugation of water-soluble polymers while minimizing the loss of biological activity associated with the modification. Consequently, polypeptides that are more highly derivatized by water-soluble polymers, and thus possessing the advantages associated with the higher degree of derivatization, may be produced that have the same level of biological activity as polypeptides derivatized by water-soluble polymers to a lesser extent.

An additional advantage of the subject invention over other methods of coupling water-soluble polymers to proteins, e. g., the use of active carbamates, is that such active esters can react with nucleophiles other than primary amines such as hydroxyl groups, phenolic groups, and sulfhydryl groups on a protein. The subject invention avoids this cross-reactivity problem in that the imidates selectively react with the primary amino group of the lysine residues.

As the polymer P comprises multiple identical units of varying amounts, it will be appreciated that the molecular weight of P may vary considerably. Furthermore, when P is said to have a given molecular weight, that molecular weight may only be approximate, reflecting the average molecular weight of a population of molecules P differing with respect to one another in regards to the number of subunits present in the molecule. In general, P will have a molecular weight of about 200 to 200,000, preferably in the range of 700 to 30,000. Suitable molecular weights for P, when the molecules of formulas (I) and (II) are to be coupled to a polypeptide will vary in accordance with the specific protein to modified.

The water-soluble polymer reagents of the subject invention may be used to modify a variety of polypeptides or similar molecules that contain primary amines on the $NH_2$ terminal amino acid and on the epsilon amines of lysine residues within the polypeptide. Polypeptides of interest include: antibodies, monoclonal and polyclonal; cytokines, including, M-CSF, GM-CSF; lymphokines, IL-2, IL-3, growth factors, including, PDGF, EGF; peptide hormones, including, hGH, erythropoietin; blood clotting factors, including, Factor VIII; immunogens; enzymes; enzyme inhibitors; ligands and the like. Polypeptides of interest may be isolated from their natural sources, genetically engineered cells, or produced by various in vitro synthesis methods.

While the water-soluble polymer reagents of the subject invention may be used to modify most polypeptides, it is of particular interest to modify (1) polypeptides for use as drugs, and (2) polypeptides for use in assays. Polypeptide for use in assays include specific binding proteins, polypeptides recognized by specific-binding proteins, and enzymes. By specific-binding proteins it is intended antibodies, hormone receptors, lectins, and the like.

Individual polypeptide molecules may be derivatized by one or more different water-soluble polymers by means of reaction with different embodiments of the compounds of formulas (I) and (II). Individual polypeptides may be modified by up to n water-soluble polymers, where n is the number of free primary amines on the polypeptide, the primary amine being from the lysines and the amino terminus.

Salts of any of the macromolecules described herein, e.g., polypetides, water-soluble polymers and derivatives thereof, will naturally occur when such molecules are present in (or isolated from) aqueous solutions of various pHs. All salts of peptides and other macromolecules having the indicated biological activity are considered to be within the scope of the present invention. Examples include alkali, alkaline earth, and other metal salts of carboxylic acid residues, acid addition salts (e.g., HCl) of amino residues, and zwitterions formed by reactions between carboxylic acid and amino residues within the same molecule.

It is also at interest to supply the water-soluble polymer reagents of formulas (I) and (II) in the form of a kit, so as to provide for the convenient and reproducible derivatization of polypeptides of interest. Kits of interest may contain solutions comprising the water-soluble polymer reagent of formulas (I) and (II), buffers, reaction indicator compounds, instruction, protein concentration measurement reagents, e.g., for Bradford assays, and the like. Reagent solutions will preferably be supplied in premeasured amounts.

## SYNTHESIS OF WATER SOLUBLE POLYMER IMIDATES

Several methods exist for the synthesis PEG-imidates. The methods used to synthesize PEG imidates may similarly be used for the synthesis of other water-soluble polymer imidates.

A preferred method of synthesizing compounds of formulas (I) and (II) involves the conversion of a PEG-alcohol to an activated alcohol that can be reacted with an appropriate nucleophile yielding a PEG-nitrile. The

PEG-nitrile then is converted to PEG-imidate. Activated PEG-alcohols may include chloride, bromide, tresyl, tosyl, and the like. References relating to the synthesis of the activated PEG-alcohol include: Furukawa, S., Katayama, N., Iizuka, T., Urabe, I., and Okada, H. (1980) FEBS Lett.: 121, 239-242; Mutter, M. (1978) Tetrahedron Lett. 2839-2842; Harris, J.M., Yalpani, M., Van Alstine, J.M., Struck, E.C., Case, M.G., Paley, M.S., and Brooks, D.E. (1984) J. Polym. Sci. Polym. Chem. Ed. 22, 341-352; Zalipsky, S., Gilon, C., and Zilkha, A. (1983) Eur. Polym. J. 19: 1177-1183; Buckmann, A.F., Kula, M.R., Wichmann, R., and Wandrey, C. (1981) J. Appl. Biochem. 3: 301-315; Buckman A.F., Morr, M., and Johansson, G. (1981) Makromol. Chem. 82: 1379-1384.

$$\text{PEG-OH} \xrightarrow{\text{4-toluenesulfonyl chloride}} \text{PEG-OTs}$$

$$\text{PEG-OTs} \xrightarrow{H_2N-(CH_2)_5-CN} \text{PEG-NH-}(CH_2)_5\text{-CN}$$

$$\text{PEG-NH-}(CH_2)_5\text{-CN} \xrightarrow{\text{HCl/MeOH}} \text{PEG-NH-}(CH_2)_5\text{-}\overset{\overset{OCH_3}{|}}{C}=NH_2^{+}\ Cl^{-}$$

A preferred method of modifying polypeptides or proteins with compounds of formulas (I) and (II) involves placing the polypeptide or protein in a basic buffer not containing a primary amine and preferably at a pH of 9-9.5. The compound of formulas (I) and (II) is added either as a single addition or multiple additions as required for the desired amount of modification.

The invention having been described, the following examples are offered to illustrate the subject invention by way of illustration, not by way of limitation.

## Examples

### I. Synthesis of PEG-imidates

PEG-CN or PEG-NH-$(CH_2)_5$-CN (0.5 g) was dissolved in 0.5 ml dry dichloromethane. Dry methanol (5 ml) was placed in a -20°C ice bath, and about 2 ml HCl was condensed into the methanol. The PEG-NH-$(CH_2)_5$-CN solution was added to the MeOH/HCl. The reaction proceeded for 5 hr at -20°C, after which time the reaction mixture was placed in the freezer overnight. The PEG-imidate was precipitated from the reaction mixture on addition of dry ether. The IR of the product showed the characteristic imidate band at 1650.

The abbreviation "PEG" as used in all of the examples in this application stands for the monomethyl ether of polyethylene glycol.

### II. Synthesis of PEG-nitrile

PEG-OH was dried for about five hr at 85°C in a high vacuum oven. After cooling 15 g PEG-OH (MW = 5000) was dissolved in 50 ml dichloromethane. Triethylamine (6.20 ml) was added followed by 11.4 g 4-toluenesulfonyl chloride. (Alternatively, 2,2,2-trifluoroethanesulfonyl chloride could be used to form a tresyl-PEG.) The mixture was refluxed overnight. Repeated precipitations with ether from dichloromethane gave the desired product PEG-OTs. Yield 14 g. Analysis. Calcd. for S, 0.63. Found: S, 0.58.

PEG-OTs (5 g, 1 mmole) was placed in 65 ml dry tetrahydrofuran along with 1.38 g (13 mmole) $Na_2CO_3$ and 0.51 g (4.6 mmole) 6-aminocapronitrile. The mixture was heated to reflux for three days. The mixture was cooled and filtered. The precipitate was dissolved in dichloromethane and filtered. Ether was added to the filtrate to precipitate the product. The precipitation step was repeated. Further purification was preformed by gel filtration on a LH-20 column eluting with MeOH/$H_2O$ (5:1). The material of interest PEG-NH-$(CH_2)_5$-CN, was collected, taken-up in "dry" dichloromethane, and precipitated on addition of ether. Yield 4.06 g. Analysis. Calcd. for N, 0.55. Found: N, 0.41.

PEG nitrile may also be made from PEG-Cl by displacement with cyanide. In this instance there is no linker

between the imidate and PEG.

$$PEG\text{-}OH \xrightarrow{SOCl_2} PEG\text{-}Cl$$

$$PEG\text{-}Cl \xrightarrow{NaCN} PEG\text{-}CN$$

PEG-OH was dried for about five hr at 85°C in a high vacuum oven. After cooling 10 g (5 mmole) PEG-OH (MW = 2000) was placed in 40 ml dry toluene followed by 0.7 ml (5 mmole) triethylamine. Thionyl chloride (1.1 ml, 5 mmole) was added slowly over a 1 hr period. The solution was refluxed for 4 hr after which time the solution was stirred over night at room temperature. The solution was heated and filtered. The filtrate was concentrated to dryness. The residue was dissolved in water and treated with activated carbon. After filtering off the carbon and concentrating the filtrate, the residue was taken up in dichloromethane and treated with ether. The precipitate was collected. Yield 8.3 g. Further purification was achieved by gel filtration chromatography on LH-20 eluting with MeOH/$H_2O$ (5:1). IR showed a chloride band at 668 for the product. Analysis. Calcd. for Cl, 1.76. Found: Cl, 1.55.

PEG-Cl (MW = 2000) 1 g (0.5 mmole) and NaCN (0.21 g, 4.2 mmole) was added to 6.6 ml dry dimethylsulfoxide. The mixture was heated at 100°C for 70 min. After cooling the solution was filtered, and the filtrate was concentrated. The residue was gel filtered on a LH-20 column eluting with MeOH/$H_2O$ (5:1). PEG-CN Yield 0.84 g. IR showed no chloride band. Analysis. Calcd. for N, 0.69. Found: N, 0.35.

III. Labeling OKT3 (Imidate Method)

OKT3 is an $IgG_{2a}$ murine monoclonal antibody that reacts with a 20,000 dalton protein associated with the human T cell antigen receptor. 4 mg OKT3 was placed in 4 ml 0.1M borate buffer, pH 9.2. The amount of PEG-imidate added to the reaction was varied in proportion with the degree of modification desired. Usually 15 mg was added; and after 2 hrs, another 15 mg PEG-imidate was added if desired. After 4 hr at room temperature reaction mixture was placed in the cold room overnight. PEG-OKT3 was separated from unreacted PEG by either hydrophobic interaction chromatography (HIC) chromatography or repeated washings/concentrations using a 30,000 MW cut-off membrane. The amount of substitution was determined by HPLC gel filtration using either a Zorbax™ GF-450 or Zorbax™ GF-250 column. Free amino groups measurement employed the fluorometric assay method of Stocks, S.J., Jones, A.J.M., Ramey, C.W., and Brooks, D.E. (1986) Anal. Biochem. 154: 232-234].

IV. Labeling OKT3 (N-Hydroxysuccinimide ester Method)

The same procedure as above for PEG-imidate was followed except PEG-N-hydroxysuccinimde was substituted for PEG-imidate.

V. Biological Activity of Labeled OKT3

The biological activities of OKT3 molecules modified by PEG-imidate and PEG-N-hydroxysuccinimide were compared. OKT3 possesses potent mitogenic properties [Van Wauwe, J.P., De Mey, J.R., and Goossens, J.G. (1980) J. Immunol. 124, 2708-2713]. To determine the effect of PEG modification on biological activity and to compare two different methods of coupling PEG to the antibody with respect to biological activity, a mitogen assay was performed. The assay consists of isolating human peripheral T lymphocytes and incubating these cells at 2 X $10^6$ cells/ml with different concentrations of OKT3 and PEG-OKT3 for four days. Then tritiated thymidine is added to the cell culture. After an overnight incubation the cells are harvested and counted.

TABLE I

Relative Mitogenic Activity (%)

| % Lysines Modified* | # PEGs Incorporated* | Im-5** | NHS-5** | Im-2** | NHS-2** |
|---|---|---|---|---|---|
| 0 | 0 | 100 | 100 | 100 | 100 |
| 20 | 17 | 30 | 30 | -- | -- |
| 30 | 26 | 30 | 2 | -- | -- |
| 45 | 39 | 1 | 0.02 | 10 | 1 |
| 60 | 52 | -- | -- | -- | 0.1 |
| 70 | 60 | -- | -- | 10 | 0.001 |

* estimated from analytical HPLC gel filtration/free amine analysis.

** Where Im-5 is imidate coupling PEG5000, NHS-5 is N-hydroxysuccinimide coupling PEG5000, Im-2 is imidate coupling PEG2000, and NHS-2 is N-hydroxysuccinimide coupling PEG2000.

The difference in mitogenic activity between imidate-PEG coupling to OKT3 and N-hydroxyssucinimide-PEG coupling to OKT3 is shown table I. At moderate to high levels, i.e., 30-7090, of modification there is considerably more mitogenic activity associated with imidate-PEG modified OKT3 than succinimide-PEG modified OKT3. Thus the preservation of the positive charge on modification as demonstrated by the imidate-PEG coupling leads to better retention of biological activity than the conventionally used succinimide-PEG (no charge preservation on modification) as shown for PEG molecular weights of 2000 and 5000.

VI. PEG-ERYTHROPOIETIN

Recombinantly produced human erythropoietin (EPO) was modified with imidate-PEG, succinimide-PEG, and tresyl-PEG. The modifications using imidate-PEG and succinimide-PEG were conducted at pH 8.2 or 9.3. Modifications with tresyl-PEG were performed at either pH 7.5, 8.2, or 9.3.

In a typical experiment, EPO (0.5 mg) was placed in 0.8 ml 0.05 M phosphate buffer/0.5 M NaCl, pH 7.5 or 0.8 ml 0.1 M borate buffer, pH 9.3. In some cases the buffers contained 0.1% SDS. 10 mg PEG with the appropriate activating group was added to the EPO solution. The reaction proceeded for about 2 hr, after which time another addition(s) of similarly activated PEG was added if there was need to increase the amount of modification on EPO. The PEG-EPO derivatives were analyzed by HPLC gel filtration using a Zorbax™ GF-250 column eluting with 0.1 M phosphate buffer, pH 7.

It was found that by using PEG-2000 (PEG with a molecular weight of 2000) a single major protein peak could be detected on analytical HPLC. If PEG-5000 is used with the three different linkers, a significant amount of unreacted EPO remained, and two major peaks representing modified EPO were detected. Thus the use of PEG-2000 resulted in a cleaner reaction product than with PEG-5000.

The EPO-PEG derivatives were purified by gel filtration using a SephacrylS-200-HR column (1mm x 45mm) eluted with a phosphate buffer (containing a small amount of azide).

The PEG-EPO derivatives were assayed for biological activity using a murine cell line which expresses the EPO receptor. This cell line is dependent on EPO for growth. The assay is as follows. The cells ($10^6$/ml) are grown in the absence of EPO for 24 hr after which time either EPO or PEG-EPO is added. The cells are incubated for 42 hr, and then tritiated thymidine is added to the cells. After 6 hr the cells are harvested and counted. Cell growth is determined by the increased uptake of thymidine. The results are shown in Table II.

TABLE II

Relative Cell Proliferation Activity of EPO and PEG-EPOs

| Linker* | #PEG-2000** | Activity (%)*** |
|---|---|---|
| EPO | 0 | 100 |
| Im | 5 | 50 |
| Im | 8 | 20 |
| Trs | 5 | 5 |
| NHS | 5 | 2 |
| NHS | 8 | 0.5 |

* where Im = imidate, Trs = tresyl, NHS = succinimide activated PEG.

** where #PEGs is the average amount of PEG (molecular weight 2000) molecules attached to a single molecule of EPO.

*** where activity is the amount of EPO or PEG-EPO required to give maximum or half-maximal cell proliferation based on EPO being 100% active.

TABLE III

Relative Cell Proliferation Activity of EPO and PEG-EPOs

| Linker* | #PEG-5000** | Activity (%)*** |
|---|---|---|
| EPO | 0 | 100 |
| Im | 5 | 15 |
| Trs | 3 | 20 |
| Trs | 5 | 3 |
| NHS | 8 | 0.1 |

* where Im = imidate, Trs = tresyl, NHS = succinimide, activated PEG.

** where #PEGs is the average amount of PEG (molecular weight 5000) molecules attached to a single molecule of EPO.

*** where activity is the amount of EPO or PEG-EPO required to give maximum or half-maximal cell proliferation based on EPO being 100% active.

As shown in Tables II and III, imidate-PEG with either 5 or 8 PEGs attached shows considerably more activity than PEG activated with either tresyl or succinimide groups. Possibly accounting for the increased biological activity of the imidate-PEG as compared with its counterparts is the preservation of the positive charge upon modification. Also of possible importance is that the positive charge is slightly displaced from the PEG backbone and thus is not as shielded by PEG and is more accessible for interaction with the receptor.

In a study comparing four different PEG coupling chemistries (tresyl, succinimidyl succinate, cyanuric chloride, and carbonyl diimidazole) on enzymatic activity of PEG-modified alkaline phosphatase, it was found that succinimidyl succinate gave the most active enzyme on modification [Yoshinga, K. and Harris, J.M. J. Bioact. Compatible Polym. 4: 17-24 (1989)]. Modification of asparaginase with PEG also showed that succinimidyl succinate yields significantly less enzymatic deactivation on coupling than PEG-cyanuric chloride [Abuchowski, A., Kazo, G.M., Verhoest, C.R., Es. T.V., Kafkewitz, D., Nucci M.L., Viau, A.T., and Davis, F.F. Cancer Biochem. Biophys. 7: 175-186 (1984)]. In both the present EPO and OKT3 studies the imidate coupling chemistry was better than succinimidyl succinate coupling chemistry, thus showing that PEG-derivatives taught herein are superior to the previously delivered best coupling PEG-chemistry.

The results in Tables II and III concerning the activity of the EPO derivatives produced from the succinimide-PEG and the tresyl-PEG esters are of interest. Although the tresyl-PEG and succinimide-PEG EPO derivatives display less activity than the comparable imidate-PEG EPO derivatives, the tresyl and succinimide-PEG derivatives display more activity than would be expected for EPO-PEG derivatives in which the coupling to lysine residues results in the loss of a positive charge (the tresyl-PEG giving rise to a secondary amine and the succinimide-PEG giving rise to an amide linkage). Thus it may be of interest to derivatize EPO (either human or non-human, isolated from natural sources, produced from genetically engineered cells, or synthesized by various in vitro methods) with tresyl or succinimide esters of water-soluble polymers (water-soluble polymers being defined the same as for P in compound (I)).

The foregoing written specification is considered to be sufficient to enable one skilled in the art to practice the invention. Indeed, various modifications of the above-described modes for carrying out the invention which are obvious to those skilled in the field of pharmaceutical formulation or related fields are intended to be within the scope of the following claims.

## Claims

1. A compound according to the formula (I)

(I)

$$\begin{array}{c} OCH_3 \\ P\text{-}C{=}NH_2^{+}Cl_{-} \end{array}$$

wherein P is a water-soluble polymer.

2. A compound according to the formula (II)

(II)

$$\begin{array}{r} OCH_3 \quad\quad \\ P\text{-}NH\text{-}(CH_2)_m\text{-}C{=}NH_2^{+}Cl^{-} \end{array}$$

wherein P is a water-soluble polymer, and m is 1 to 20.

3. The compound of Claim 1 or Claim 2, wherein said polymer P is a polyethylene glycol homopolymer, a polypropylene glycol homopolymer, a copolymer of ethylene glycol with propylene glycol, wherein said homopolymer or said copolymer is unsubstituted or substituted at one end with an alkyl group, a polyoxyethylated polyol, a polyvinyl alcohol, a polysaccharide, a polyvinyl ethyl ether, or α,β-poly[(2-hydroxyethyl)-DL-aspartamide].

4. The compound of Claim 3, wherein said water-soluble polymer is polyethylene glycol.

5. A method a covalently joining at least one water-soluble polymer to a polypeptide, said method comprising the step of reacting a compound according to any one of Claims 1 to 4 with a polypeptide.

6. The method of Claim 5, wherein said polypeptide is a hormone, a lymphokine, a cytokine, a growth factor, an enzyme, a vaccine antigen, or an antibody.

7. The method of Claim 6, wherein said polypeptide is an antibody, and said method further comprises the

step of combining said antibody with a compound capable of specifically binding to a binding site on said antibody, prior to said reacting step.

**8.** The method of Claim 6, wherein said polypeptide is an enzyme, and said method further comprises the step of mixing said polypeptide with a substrate for said enzyme, prior to said reacting step.

**9.** A polypeptide modified by the method of any one of Claims 5 to 8.

**10.** A composition comprising the polypeptide of Claim 9, and a pharmaceutically acceptable carrier.

**11.** A compound of the formula (III)

$$\text{(III)} \qquad \begin{array}{c} \quad NH_2Cl_ \\ [P-C-NH-]_n-Z \end{array}$$

wherein n is 1 to x, x being one larger than the number of lysine residues in Z, P is a water-soluble polymer, and Z is a polypeptide, wherein Z is covalently joined to the non-polypeptide portion of said compound through a lysine residue in Z.

**12.** A compound of the formula (IV)

$$\text{(IV)} \qquad \begin{array}{c} \qquad\qquad NH_2{}^+Cl^- \\ [P-NH-(CH_2)_m-C-NH-]_n-Z \end{array}$$

wherein m is 1 to 20, n is 1 to x, x being one larger than the number of lysine residues in Z, P is a water-soluble polymer, and Z is a polypeptide, wherein Z is covalently joined to the non-polypeptide portion of said compound through a lysine residue in Z.

**13.** The compound of Claim 11 or Claim 12, wherein said polymer is a polyethylene glycol homopolymer, a polypropylene glycol homopolymer, a copolymer of ethylene glycol with propylene glycol, wherein said homopolymer or copolymer is unsubstituted or substituted at one end with an alkyl group, a polyoxyethylated polyol, a polyvinyl alcohol, a polysaccharide, a polyvinyl ethyl ether, or α,β-poly[(2-hydroxyethyl)-DL-aspartamide].

**14.** The compound of any one of Claima 11 to 13, wherein said polypeptide is a hormone, a lymphokine, a cytokine, a growth factor, an enzyme, a vaccine antigen, or an antibody.

**15.** The compound of Claim 14, wherein said polypeptide is an antibody.

**16.** The compound of Claim 14, wherein said polypeptide is erythropoietin.

**17.** A composition comprising the compound of any one of Claims 11 to 16, and a pharmaceutically acceptable carrier.

**18.** A compound according to the formula (V)

$$\text{(V)} \qquad P-NH-(CH_2)_n-CN$$

wherein P is a water-soluble polymer and n = 1-20.

**19.** The compound of Claim 18, wherein said polymer is a polyethylene glycol homopolymer, a polypropylene glycol homopolymer, a copolymer of ethylene glycol with propylene glycol, wherein said homopolymer or copolymer is unsubstituted or substituted at one end with an alkyl group, a polyoxyethylated polyol, a polyvinyl alcohol, a polysaccharide, a polyvinyl ethyl ether, or α,β-poly[(2-hydroxyethyl)-DL-aspartamide].

**20.** The compound of Claim 19, wherein said water-soluble polymer is polyethylene glycol.

**21.** An erythropoietin molecule modified by at least one tresyl water-soluble polymer ester, wherein said water

soluble polymer is a polyethylene glycol homopolymer, a polypropylene glycol homopolymer, a copolymer of ethylene glycol with propylene glycol, wherein said homopolymer or copolymer is unsubstituted or substituted at one end with an alkyl group, a polyoxyethylated polyol, a polyvinyl alcohol, a polysaccharide, a polyvinyl ethyl ether, or α,β-poly[2-hydroxyethyl)-DL-aspartamide].

**22.** An erythropoietin molecule modified by at least one succinimide water-soluble polymer ester, wherein said water soluble polymer is a polyethylene glycol homopolymer, a polypropylene glycol homopolymer, a copolymer of ethylene glycol with propylene glycol, wherein said homopolymer or copolymer is unsubstituted or substituted at one end with an alkyl group, a polyoxyethylated polyol, a polyvinyl alcohol, a polysaccharide, a polyvinyl ethyl ether, or α,β-poly[(2-hydroxyethyl)-DL-aspartamide].

**23.** The erythropoietin molecule of Claim 21 or Claim 22, wherein said water-soluble polymer is polyethylene glycol.